**Europäisches Patentamt**

**European Patent Office**

**Office européen des brevets**

(19)

(11) Veröffentlichungsnummer: **0 182 385
B1**

(12) ## EUROPÄISCHE PATENTSCHRIFT

(45) Veröffentlichungstag der Patentschrift:
**03.05.89**

(51) Int. Cl.⁴: **G 01 N 33/78**

(21) Anmeldenummer: **85114863.5**

(22) Anmeldetag: **22.11.85**

(54) Verfahren und Reagenz zur quantitativen Bestimmung von freiem Thyroxin in Plasma, Serum oder Vollblut.

(30) Priorität: **23.11.84 DE 3442817**

(43) Veröffentlichungstag der Anmeldung:
**28.05.86 Patentblatt 86/22**

(45) Bekanntmachung des Hinweises auf die Patenterteilung:
**03.05.89 Patentblatt 89/18**

(84) Benannte Vertragsstaaten:
**AT BE CH DE FR GB IT LI LU NL SE**

(56) Entgegenhaltungen:
**EP-A- 0 073 513
DE-A- 3 136 579
US-A- 4 366 143**

(73) Patentinhaber: **BOEHRINGER MANNHEIM GMBH,
Patentabteilung, Abt. E Sandhofer
Strasse 112-132 Postfach 31 01 20,
D-6800 Mannheim 31 Waldhof (DE)**

(72) Erfinder: **Berger, Johann, Traubinger Strasse 35,
D-8132 Tutzing (DE)**
Erfinder: **Jering, Helmut, Anton-Bartl-Strasse 4,
D-8132 Tutzing (DE)**

(74) Vertreter: **Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)**

**Beschreibung**

Thyroxin ($T_4$) ist nach den derzeitigen Kenntnissen zu ungefähr 80% an TBG (Thyroxin bindendes Globulin) zu ungefähr 15% an TBPA und zu ungefähr 5% an Albumin gebunden. Als Bindungskonstanten werden zirka $2 \times 10^{10}$ 1/Mol für TBG, zirka $10^6$ bis $10^8$ für TBPA und $10^5$ bis $10^6$ für Albumin angegeben. Ein sehr geringer Teil des $T_4$ liegt «frei», d.h. in nicht an Protein gebundener Form, vor und wird als «$FT_4$» bezeichnet. $FT_4$ liegt offenbar in einer Menge von zirka 0,01 bis 0,03%, bezogen auf die gesamte $T_4$-Menge, vor. Bei einem $T_4$-Normalbereich von 100 nmol/l entspricht das einer Konzentration von zirka 10 pg/ml bzw. 15 pmol/l im Normalbereich. Verschiedentlich wird angenommen, daß nur $FT_4$ tatsächlich physiologisch wirksam ist, da es die Zellmembranwand passieren kann, im Gegensatz zum proteingebundenen $T_4$. $FT_4$ steht dann im Blut im Gleichgewicht mit dem proteingebundenen $T_4$, das als Puffer in erster Linie zur Regelung des $FT_4$-Spiegels dient.

Die Konzentration an $FT_4$ reflektiert daher den funktionellen Thyroidstatus unabhängig von Änderungen in der Konzentration oder Sättigung von Thyroxin bindendem Protein und ist infolgedessen klinisch bedeutsam.

Die anerkannte Bezugsmethode zur Bestimmung von $FT_4$ beruht auf einer Gleichgewichts-Dialyse von Serum, welches mit radioaktiv markiertem $T_4$ angereichert war, oder auf einem Radioimmunoassay vom Dialysat ohne vorherige Anreicherung mit radioaktiv markiertem $T_4$. (J. Clin. Immunoassay, Vol. 7, 1984, 192–205).

Diese Methode wird als zuverlässig angesehen, scheidet jedoch aufgrund des sehr hohen Zeitbedarfs für die Routinebestimmung (12 Stunden Inkubation, 18 Stunden Dialyse) aus.

Ebenfalls wurde bereits vorgeschlagen, eine Extraktion mit Anti-$T_4$-Antikörpern durchzuführen oder $FT_4$ rechnerisch zu ermitteln durch Multiplikation von Gesamt-$T_4$ mit dem sogenannten $T_3$-Aufnahme oder durch Gesamt-$T_4$ multipliziert mit 1/TBG zu errechnen in der Annahme, daß die so erhaltenen Werte unter den meisten Umständen mit der Konzentration an $FT_4$ korrelieren. (J. of Clin. Immunoassay, Vol. 7, 1984, 192–205). Eine weitere Methode beruht auf der Verwendung eines $T_4$-analogen Tracers, der zwar von Anti-$T_4$-Antikörpern gebunden werden soll, nicht aber von den Serumproteinen (USP 4 366 143). Die Richtigkeit und Aussagekraft dieser Verfahren wird jedoch angezweifelt (Clin. Chemistry, 30, 1984, 491–493, J. of Clin. Immunoassay, Vol. 7, 1984, 192–205).

Der Erfindung liegt daher die Aufgabe zugrunde, ein einfach durchzuführendes Verfahren mit hoher Genauigkeit zu schaffen, welches sich insbesondere auch für die Automatisierung eignet.

Diese Aufgabe wird erfindungsgemäß gelöst durch ein Verfahren zur quantitativen Bestimmung von freiem Thyroxin ($FT_4$) in Plasma, Serum oder Vollblut nach immunologischen Methoden, welches dadurch gekennzeichnet ist, daß man die Probe höchstens 10 Minuten bei einem 10- bis 2000fachen Unterschuß an markiertem Anti-$T_4$-Antikörper, bezogen auf die molare Menge an Gesamt-$T_4$ in der Probe, inkubiert, dann sofort mit überschüssigem immobilisiertem $T_4$ zusammenbringt, erneut inkubiert, die Phasen trennt und in einer der Phasen die Markierung mißt.

Durch die Kombination eines sehr großen Unterschusses an markiertem Anti-$T_4$-Antikörper und einer sehr kurzen Inkubationszeit bis zum Kontakt mit überschüssigem immobilisiertem $T_4$ gelingt es überraschenderweise, die außerordentlich geringen Mengen an $FT_4$ sehr genau zu bestimmen.

Die Inkubationsdauer der Probe mit dem in starkem Unterschuß vorliegenden markierten Anti-$T_4$-Antikörper soll möglichst gering gehalten werden und wird daher vorzugsweise nur ein bis fünf Minuten durchgeführt. Noch kürzere Inkubationszeiten läßt das erfindungsgemäße Verfahren ebenfalls zu und eine Inkubationsdauer im Bereich von 10 bis 30 Sekunden ist für das erfindungsgemäße Verfahren immer noch brauchbar, von der Handhabung her jedoch nicht leicht zu erreichen.

Der Unterschuß an markiertem Anti-$T_4$-Antikörper, der auf die molare Menge an Gesamt-$T_4$ in der Probe bezogen wird, entspricht vorzugsweise der prozentualen Menge an $FT_4$, bezogen auf Gesamt-$T_4$. Als besonders geeignet hat sich ein 10- bis 2000-facher Unterschuß, vorzugsweise ein 50- bis 2000-facher Unterschuß erwiesen. Der markierte Anti-$T_4$-Antikörper kann jedoch auch in einem Unterschuß oder Überschuß gegenüber $FT_4$ eingesetzt werden. Bei einem Überschuß empfiehlt es sich, im unteren Zeitbereich für die erste Inkubation zu arbeiten, bei einem Unterschuß kann auch die obere Zeitgrenze ausgenutzt werden.

Die zweite Inkubation mit überschüssigem immobilisiertem $T_4$ unterliegt keinen zeitlichen Beschränkungen der Obergrenze, jedoch sollte in der Regel eine Minute nicht unterschritten werden. Ein bis zehn Minuten sind in der Regel geeignet, wesentlich längere Inkubationszeiten können zwar angewendet werden, bringen aber keinen Vorteil. Vier bis sechs Minuten Inkubation ergaben in der Praxis sehr gute Ergebnisse. Diese Inkubationszeiten eignen sich auch gut für die Durchführung des Verfahrens auf Analysenautomaten.

Als Antikörper wird vorzugsweise ein solcher verwendet, dessen Affinität gleich oder kleiner der Komplexbildungskonstanten des in der Probe vorhandenen gebundenen $T_4$ ist. Besonders bevorzugt verwendet man Anti-$T_4$-Antikörper mit einer Affinitätskonstante von $10^{10}$ l/Mol oder kleiner.

Der Anti-$T_4$-Antikörper kann in der in immunologischen Tests bekannten Weise markiert sein. Geeignete Markierungen können z.B. durch kovalente Verknüpfung mit einem gut bestimmbaren Enzym wie Peroxidase, β-Galactosidase und dergleichen, durch optisch bestimmbare Liganden wie fluoreszierende, phosporeszierende und ähnliche Substanzen oder durch radioaktive Markierung erfolgen. Diese Methoden sind dem Fachmann bekannt und bedürfen hier keiner näheren Erläuterung. Bevorzugt wird eine Enzymmarkierung.

Das in fester Phase vorliegende immobilisierte $T_4$ muß, wie erwähnt, im Überschuß vorliegen. Bevorzugt wird ein Überschuß, der etwa der 10- bis 5000-fachen Menge, bezogen auf die eingesetzte molare Menge an markiertem Anti-$T_4$-Antikörper, entspricht, besonders bevorzugt wird ein 100- bis 1000-facher Überschuß. Wesentlich größere Mengen können verwendet werden, sie verbessern aber das Ergebnis nicht, erhöhen jedoch die Kosten. Eine Unterschreitung bis herab zu einem etwa fünffachen Überschuß ist ebenfalls möglich, jedoch empfiehlt es sich dann, die Dauer der zweiten Inkubation zu erhöhen.

Die Immobilisierung des $T_4$ kann beispielsweise nach den üblichen Immobilisierungsmethoden erfolgen, von denen kovalente Bindung an eine reaktive Matrix, Kupplung über difunktionelle Brückenbildner, Oberflächenadsorption und Oberflächenvernetzung mit Vernetzungsmitteln wie Glutardialdehyd und immunologische Fällung genannt seien.

Als Träger können alle inerten festen Trägermaterialien verwendet werden, welche eine ausreichende Bindung von $T_4$ ermöglichen und andererseits keine schädlichen Nebenwirkungen auf die Reaktion ausüben. Im übrigen sind Form und Material des Trägers wenig wichtig. Bevorzugt werden flächige Träger wie Vliese, Schaumstoff-Folien und dergleichen verwendet, besonders bevorzugt wird Papier.

Die Inkubationsdauer der ersten Stufe des erfindungsgemäßen Verfahrens entspricht der Zeit zwischen dem Zusammengeben von Probe mit markiertem Anti-$T_4$-Antikörper und dem Zusammenbringen der dabei erhaltenen Mischung mit dem immobilisierten $T_4$. Die zweite Inkubation beginnt mit dem Ende der ersten Inkubation und endet mit der Phasentrennung. Für die Einhaltung der vorgegebenen kurzen Inkubationszeiten können die dem Fachmann geläufigen Methoden angewendet werden wie z.B. Mischen von Probe und Antikörperlösung, Inkubation und Aufgeben der Mischung auf eine Säule mit immobilisiertem $T_4$ oder Überführung in ein Reagenzglas mit wandgebundenem $T_4$. Bevorzugt erfolgt die Trennung und Überführung durch Zentrifugieren, wofür sich besonders das in der EP-A 073513 beschriebene Verfahren und die dort beschriebene Vorrichtung eignet.

Durch die Erfindung wird erreicht, daß die Bestimmung von $FT_4$ auf einfachste Weise in sehr kurzer Zeit mit hoher Genauigkeit durchgeführt werden kann und damit ein routinemäßig und insbesondere auf Analysenautomaten durchführbares Verfahren geschaffen wird, welches es erlaubt, die diagnostische Bedeutung von $FT_4$ auch in der Praxis voll auszunützen.

Die folgenden Beispiele erläutern die Erfindung weiter.

Beispiel 1

Zur Durchführung des Verfahrens wurde die in EP-A 073513 beschriebene Methode und das in den Fig. 1a und 1b beschriebene Einsatzelement für ein Zentrifugunalanalysenautomaten verwendet. Dieses Einsatzelement enthält sieben miteinander verbundene Kammern, die jeweils ein Vlies enthalten und nacheinander unter dem Einfluß der Zentrifugalkraft von Flüssigkeit passiert werden.

Folgende Vliese und Tränklösungen für die Vliese wurden angewendet:

Vlies 1:         Filterpapier
Tränklösung:   3‰ Netzmittel (Tween 20)®.

Vlies 2:         Filterpapier
Tränklösung:   100 mmol Natrimphosphatpuffer pH 7,2
                   5 mmol EDTA
                   1% Rinderserumalbumin
                   0,75‰ Netzmittel Tween 20®.

Vlies 3:
Verwendetes Papier: Filterpapier
Verwendete Tränklösung:
Anti-$T_4$-Antikörper vom Schaf, markiert mit β-Galactosidase
100 mU/ml Aktivität bestimmt mit o-Nitrophenylgalactosid als Substrat
1% Rinderserumalbumin
4 mmol Magnesiumaspartat
50 mmol Hepes-Puffer pH 7,2.

Vlies 4:
Filterpapier mit BrCN aktiviert und mit $T_4$ umgesetzt.

Vlies 5:
Papier: Filterpapier
Tränklösung: 15mM Chlorphenolrot-Galactosid (CPRG) (hergestellt nach DE 3345748)

Die Kammern des Einsatzelementes wurden wie folgt belegt:

Kammer I:     1 Vlies 1
Kammer II:    1 Vlies 2
Kammer III:   1 Vlies 3
Kammer V:    2 Vlies 4
Kammer VI:   1 Vlies 5

Das als Probelösung verwendete Serum wurde 1:15 mit 0,9% NACl-Lösung verdünnt. 60 µl der so erhaltenen Lösung wurde in die Probeauftragskammer des Einsatzelementes pipettiert und dann folgendes Zentrifugationsprogramm durchgeführt:

| | | |
|---|---|---|
| 25 Sekunden | 250 rpm | Ablösung von Detergens, Puffer und Konjugat; Start erste Inkubation |
| 20 Sekunden | 2000 rpm | |
| 300 Sekunden | 600 rpm | Inkubation von Probe und Anti-$T_4$-AK-Konjugat |
| 300 Sekunden | 0 rpm | (Inkubation mit trägerfixiertem $T_4$) |
| 15 Sekunden | 2000 rpm | Beendigung der zweiten Inkubation |
| 15 Sekunden | 0 rpm | |

5 Sekunden 100 rpm (Transport der Flüssigkeit in Küvette)
50 Sekunden 720 rpm Messung bei 578 nm.

Mit dem oben beschriebenen Zentrifugationsprogramm wird zuerst von Vlies 1 das Netzmittel
zur Erleichterung des Flüssigkeitstransports abgelöst. Anschließend werden Puffer und Konjugatvlies eingeweicht und die darauf befindlichen Bestandteile abgelöst. Nach 300 Sekunden Inkubation in der ersten Ventilkammer, in der sich FT$_4$
mit dem Anti-T$_4$-AK-Enzymkonjugat verbindet,
wird der nicht umgesetzte Teil an Konjugat im
nächsten Schritt in 300 Sekunden an immobilisiertem T$_4$ gebunden. Nach Überschleudern eines Vlieses, auf dem sich das Substrat (CPRG) befindet
und dabei abgelöst wird, gelangt die Flüssigkeit in
die Küvette, wo die Extinktionszunahme während
50 Sekunden verfolgt wird. Die Auswertung erfolgt über Eichkurve.
Beispiel 2
Testprinzip:
Mikrotiterplatten oder Kunststoffröhrchen werden
mit T$_4$-Polyhapten (Kupplung von T$_4$ an RSA, hergestellt nach DE 26 31 656) beschichtet. Probe wird
in einer ersten Reaktion in einem inerten Gefäß
mit Antikörper-Enzymkonjugat 1 bis 5 Minuten
inkubiert, anschließend wird diese Lösung in die
Mikrotiterplatten bzw. Kunststoffröhrchen pipettiert. Nach 5 Minuten wird die Lösung entfernt und
die Enzymaktivität entweder der Festphase oder
des entfernten Überstandes bestimmt.
Beschichtungspuffer
0,2 mol NaHCO$_3$ pH 9,5 + 0,01% Rinderserumalbumin
Beschichtung mit 200 µg/ml T$_4$-Polyhapten.

Nach 10 Minuten Inkubation bei Raumtemperatur wird abgesaugt und 10 Minuten nachbeschichtet mit 50 mmol Natriumphosphat, pH 7,2, 100
mmol NaCl, 1% RSA und wieder abgesaugt.

20 µl Probe werden mit 200 µl Puffer (50 mmol
Natriumphosphat, pH 7,2, 100 mmol NaCl, 1%
RSA) und 80 µl Puffer + 10 mU Konjugat versetzt
und 5 Minuten bei Raumtemperatur inkubiert. Anschließend werden 200 µl in die Mikrotiterplatten
bzw. Kunststofröhrchen pipettiert, und nach 5 Minuten Inkubation bei Raumtemperatur werden
200 µl abgesaugt, mit 200 µl Puffer gewaschen
und nach nochmaligem Absaugen mit 800 µl Substrat (5 mmol/l CPRG, 50 mmol Hepes, pH 7,2)
versetzt. Bei λ = 578 nm wird die Zunahme der
Absorption während fünf Minuten verfolgt. Die
Steigung ist das Meßsignal, die Konzentrationsbestimmung wird über eine Eichkurve vorgenommen.

**Patentansprüche**

1. Verfahren zur quantitativen Bestimmung von
freiem Thyroxin (FT$_4$) in Plasma, Serum oder Vollblut nach immunologischen Methoden, dadurch
gekennzeichnet, daß man die Probe höchstens 10
Minuten mit einem 10- bis 2000fachen Unterschuß an markiertem Anti-T$_4$-Antikörper, bezogen

auf die molare Menge an Gesamt-T$_4$ in der Probe,
inkubiert, dann sofort mit überschüssigem immobilisiertem T$_4$ zusammenbringt, erneut inkubiert,
die Phasen trennt und in einer der Phasen die
Markierung mißt.

2. Verfahren nach Anspruch 1, dadurch gekennzeichnet, daß man die erste Inkubation 1 bis 5
Minuten durchführt.

3. Verfahren nach Anspruch 1 oder 2, dadurch
gekennzeichnet, daß man einen Anti-T$_4$-Antikör-
per mit einer Affinitätskonstante von 10$^{10}$ l/Mol
oder kleiner verwendet.

4. Verfahren nach einem der vorhergehenden
Ansprüche, dadurch gekennzeichnet, daß man einen 10- bis 5000fachen Überschuß an immobilisiertem T$_4$, bezogen auf die Menge an markiertem
Anti-T$_4$-Antikörper, einsetzt.

5. Verfahren nach einem der vorhergehenden
Ansprüche, dadurch gekennzeichnet, daß man
markierten Anti-T$_4$-Antikörper in trockener Form
auf einem festen Trägermaterial ablösbar imprägniert oder lyophilisiert, einsetzt.

6. Verfahren nach Anspruch 5, dadurch gekennzeichnet, daß man den markierten Anti-T$_4$-Antikör-
per auf einem Trägervlies anordnet, die Probe auf
das Trägervlies bringt, nach der ersten Inkubation
abzentrifugiert und auf ein zweites Trägervlies
bringt, welches immobilisiertes, nicht ablösbares
T$_4$ enthält.

**Claims**

1. Process for the quantitative determination of
free thyroxine (FT$_4$) in plasma, serum or whole
blood by immunological methods, characterized
in that the sample is incubated for at most 10
minutes with a 10 to 2000 fold insufficiency of
labelled anti-T$_4$ antibodies, referred to the molar
amount of total T$_4$ in the sample, then immediately brought together with immobilised excess T$_4$,
again incubated, the phases separated and the
label measured in one of the phases.

2. Process according to claim 1, wherein the first
incubation is carried out for 1 to 5 minutes.

3. Process according to claim 1 or 2, wherein an
anti-T$_4$ antibody is used with an affinity constant
of 10$^{10}$ l/mole or less.

4. Process according to any of the preceding
claims, wherein there is used a 10 to 5000 fold
excess of immobilised T$_4$, referred to the amount
of labelled anti-T$_4$ antibody.

5. Process according to any of the preceding
claims, wherein there is used labelled anti-T$_4$ antibody dissolvably impregnated or lyophilised in
dry form on a solid carrier material.

6. Process according to claim 5, wherein the
labelled anti-T$_4$ antibody is provided on a carrier
fleece, the sample is applied to the carrier fleece
and, after the first incubation, it is centrifuged off
and brought on to a second carrier fleece which
contains immobilised T$_4$ which cannot be dissolved off.

**Revendications**

1. Procédé de détermination quantitative de la thyroxine (FT$_4$) libre dans le plasma, le sérum ou le sang complet par des méthodes immunologiques, caractérisé en ce qu'on fait incuber l'échantillon au plus 10 minutes avec une quantité insuffisante de 10 à 2000 fois d'anticorps anti-T$_4$ marqué, par rapport à la quantité molaire de T$_4$ totale dans l'échantillon, puis en ce qu'on mélange immédiatement avec de la T$_4$ immobilisée en excès, en ce qu'on fait incuber à nouveau, en ce qu'on sépare les phases et en ce qu'on mesure le marquage dans une des phases.

2. Procédé suivant la revendication 1, caractérisé en ce qu'on effectue la première incubation pendant 1 à 5 minutes.

3. Procédé suivant les revendications 1 ou 2, caractérisé en ce qu'on utilise un anticorps anti-T$_4$ ayant une constante d'affinité de 10$^{10}$ l/mole ou moins.

4. Procédé suivant l'une des revendications précédentes, caractérisé en ce qu'on utilise un excès de 10 à 5000 fois de T$_4$ immobilisée, par rapport à la quantité d'anticorps anti-T$_4$ marqué.

5. Procédé suivant l'une des revendications précédentes, caractérisé en ce qu'on utilise un anticorps anti T$_4$ marqué imprégné ou lyophilisé sous forme sèche sur une matière de support solide de manière à pouvoir être dissous.

6. Procédé suivant la revendication 5, caractérisé en ce qu'on dispose l'anticorps anti-T$_4$ marqué sur une nappe fibreuse de support, en ce qu'on dépose l'échantillon sur la nappe de support, en ce qu'on le centrifuge aprés la première incubation et en ce qu'on le dépose sur une deuxième nappe fibreuse de support, qui contient de la T$_4$ immobilisé, non éliminable par dissolution.